Europäisches Patentamt

European Patent Office

Office européen des brevets

(11) Publication number : **0 519 750 A2**

## (12) EUROPEAN PATENT APPLICATION

(21) Application number : **92305678.2**

(22) Date of filing : **19.06.92**

(51) Int. Cl.⁵ : **C07K 7/40, A61K 37/26**

(30) Priority : **21.06.91 US 718574**

(43) Date of publication of application :
**23.12.92 Bulletin 92/52**

(84) Designated Contracting States :
**AT BE CH DE DK ES FR GB GR IT LI LU NL PT SE**

(71) Applicant : **ELI LILLY AND COMPANY**
**Lilly Corporate Center**
**Indianapolis Indiana 46285 (US)**

(72) Inventor : **Brems, David Nettleship**
**9909 East Shahan**
**Indianapolis, Indiana 46256 (US)**
Inventor : **Chance, Ronald Eugene**
**19303 Flippin Road**
**Westfield, Indiana 46074 (US)**
Inventor : **Frank, Bruce Hill**
**9377 Spring Forest Drive**
**Indianapolis, Indiana 46260 (US)**

(74) Representative : **Hudson, Christopher Mark et al**
**Erl Wood Manor**
**Windlesham Surrey GU20 6PH (GB)**

(54) **Insulin analogs.**

㊱   Analogs of human insulin containing an Asp residue at position 10 of the B chain, having a truncated carboxyl terminus of the B chain and, optionally, having modifications at other positions display modified physico-chemical and pharmacokinetic properties. These analogs are useful in the treatment of hyperglycemia.

EP 0 519 750 A2

The present invention is in the field of human medicine, particularly in the treatment of diabetes. Most specifically, the invention relates to analogs of the human insulin molecule, methods of using these analogs and pharmaceutical compositions comprising these insulin analogs.

Diabetes mellitus is a metabolic disorder characterized by the failure of body tissues to oxidize carbohydrates at the normal rate. A deficiency of insulin is the most important factor in the diabetic disease state. During the last 70 years people suffering from diabetes have been greatly aided by receiving controlled amounts of insulin. Until the early 1980's, the insulin used by diabetics was isolated from animal pancreases, generally bovine and porcine. With the introduction of recombinant DNA technology it has become possible to produce large amounts of natural human insulin as well as naturally and non-naturally occurring analogs of human insulin. These insulin analogs display different physical and chemical properties when compared to natural human insulin.

Natural human insulin contains a histidine residue at position 10 of the B-chain. Replacement of this histidine residue with an aspartic acid residue, and the removal of the lysine and the threonine residues at positions 29 and 30 of the B-chain creates an analog Asp(B10), des(B29-30) human insulin which aggregates in solution. Surprisingly, the removal of the proline residue at position 28 of this analog to obtain Asp(B10), des(B28-30) human insulin leads to a molecule which is stable, fast-acting and is maintained in solution predominantly as a monomer. Furthermore, the elimination of the threonine residue at position 27 of the B-chain of Asp(B10), des(28-30) human insulin creates Asp(B10), des(27-30) human insulin which also is stable, fast-acting and monomeric in solution.

The present invention relates to analogs of human insulin modified by changing amino acid 10 of the native human insulin B chain from histidine to aspartic acid and by removing the tripeptide or tetrapeptide from the carboxyl terminus of the native human insulin B-chain. These molecules may also be modified at position 21 of the native human insulin A-chain and at both positions 1 and 3 of the native human insulin B-chain. Said insulin analogs are more stable and less prone to dimerization or self-association to higher molecular weight forms thereby possess a comparatively more rapid onset of activity while retaining the biological activity of native human insulin.

Also disclosed and claimed is a method of treating hyperglycemia by administering to a patient in need thereof an effective amount of the claimed analogs. Further, pharmaceutical compositions containing an effective amount of an insulin analog of the invention in combination with one or more pharmaceutically acceptable excipients are disclosed and claimed.

For purposes of the present invention, as disclosed and claimed herein, the following terms and abbreviations are as defined below.

Asp(B10), des(B28-30) HI - a human insulin analog wherein the histidine residue at position 10 of the B-chain of native human insulin has been changed to an aspartic acid residue and amino acid residues 28 through 30 of the native human insulin B-chain have been eliminated.

Asp(B10), des(B27-30) HI - a human insulin analog wherein the histidine residue at position 10 of the B-chain of native human insulin has been changed to an aspartic acid residue and amino acid residues 27 through 30 of the native human insulin B-chain have been eliminated.

BHI - biosynthetic human insulin.

Cross-link - the formation of disulfide bonds between cysteine residues. A properly cross-linked native human insulin or insulin analog contains three disulfide bridges. The first disulfide bridge is found between the cysteine residues at positions 6 and 11 of the A-chain. The second disulfide bridge links the cysteine at position 7 of the A-chain to the cysteine at position 7 of the B-chain. The third disulfide bridge links the cysteine at position 20 of the A-chain to the cysteine at position 19 of the B-chain.

SEQ ID NO:1 - the first sequence set forth in the sequence listing which is an analog of the human insulin A-chain with the sequence

```
Gly Ile Val Glu Gln Cys Cys Thr Ser Ile Cys Ser Leu Tyr Gln
 1                   5                  10                  15

Leu Glu Asn Tyr Cys Xaa
                  20
```

wherein Xaa is Asn, Asp, Glu, Gln, Ala, Gly or Ser.

SEQ ID NO:2 - the second sequence set forth in the sequence listing which is an analog of the human insulin B-chain with the sequence

```
Xaa Val Xaa Gln His Leu Cys Gly Ser Asp Leu Val Glu Ala Leu
 1               5                    10                    15


Tyr Leu Val Cys Gly Glu Arg Gly Phe Phe Tyr Thr
              20                    25
```

wherein Xaa at position 1 is Phe or Asp and Xaa at position 3 is Asn or Asp.

All amino acid abbreviations used in this disclosure are those accepted by the United States Patent and Trademark Office as set forth in 37 C.F.R. §1.822(b)(2) (1990).

The present invention relates to insulin analogs of the formula

SEQ ID NO:1

```
(Gly Ile Val Glu Glu Cys Cys Thr Ser Ile Cys Ser Leu Tyr Gln Leu Glu
  1               5                    10                    15


Asn Tyr Cys Xaa)
         20
```

properly cross-linked to SEQ ID NO:2

```
(Xaa Val Xaa Gln His Leu Cys Gly Ser Asp Leu Val Glu Ala Leu Tyr Leu
  1               5                    10                    15


Val Cys Gly Glu Arg Gly Phe Phe Tyr Thr)
        20                    25
```

or a pharmaceutically acceptable salt thereof, wherein Xaa at position 21 of SEQ ID NO:1 (insulin A-chain) is Asn, Asp, Glu, Gln, Ala, Gly or Ser; Xaa at position 1 of SEQ ID NO:2 (insulin B-chain) is Phe or Asp; and Xaa at position 3 of SEQ ID NO:2 is Asn or Asp. Preferably, Xaa at position 21 of SEQ ID NO:1 is Gly, Ala, Ser or Asn. Asparagine is the most particularly preferred amino acid at position 21. The preferred amino acid at position 1 of SEQ ID NO:2 is Phe and the most preferred residue at position 3 of SEQ ID NO:2 is Asn. A particularly preferred insulin analog of the present invention is one wherein Xaa at position 21 of SEQ ID NO:1 is Asn, Xaa at position 1 of SEQ ID NO:2 is Phe and Xaa at position 3 of SEQ ID NO:2 is Asn. In standard biochemical terms known to the skilled artisan said particularly preferred analog is Asp(B10), des (B28-30) Human Insulin.

The present invention also relates to insulin analogs of the formula

SEQ ID NO:1 properly cross-linked to amino acids 1 through 26 of SEQ ID NO:2, or a pharmaceutically acceptable salt thereof,

wherein Xaa at position 21 of SEQ ID NO:1 is Asn, Asp, Glu, Gln, Ala, Gly or Ser; Xaa at position 1 of SEQ ID NO:2 is Phe or Asp; and Xaa at position 3 of SEQ ID NO:2 is Asn or Asp. Preferably, Xaa at position 21 of SEQ ID NO:1 is Gly, Ala, Ser or Asn, with Asn being most preferred. The preferred amino acid at position 1 of SEQ ID NO:2 is Phe and the most preferred residue at position 3 of SEQ ID NO:2 is Asn. A particularly preferred analog of the present invention is one wherein Xaa at position 21 of SEQ ID NO:1 is Asn, Xaa at position 1 of SEQ ID NO:2 is Phe, Xaa at position 3 of SEQ ID NO:2 is Asn, and SEQ ID NO:2 ends at residue 26. In standard biochemical terms said particularly preferred analog is Asp(B10), des(B27-30) Human Insulin.

Another analog of the present invention has the formula

SEQ ID NO: 1 properly cross-linked to amino acids 2 through 27 of SEQ ID NO:2, or a pharmaceutically acceptable salt thereof,

wherein Xaa at position 21 of SEQ ID NO: 1 is Asn, Asp, Glu, Gln, Ala, Gly or Ser; and Xaa at position 3 of SEQ ID NO:2 is Asn or Asp. Preferably, Xaa at position 21 of SEQ ID NO: 1 is Asn and Xaa of position 3 of

3

SEQ ID NO:2 is also Asn. Said preferred analog is known to the skilled artisan as Asp(B10), des(BI), des(B28-30) Human Insulin.

Yet another analog of the present invention has the formula

SEQ ID NO:1 properly cross-linked to amino acids 2 through 26 of SEQ ID NO:2, or a pharmaceutically acceptable salt thereof,

wherein Xaa at position 21 of SEQ ID NO:1 is Asn, Asp, Glu, Gln, Ala, Gly or Ser; and Xaa at position 3 of SEQ ID NO:2 is Asn or Asp. Preferably, Xaa at position 21 of SEQ ID NO:1 is Asn and Xaa at position 3 of SEQ ID NO:2 is also Asn. Said preferred analog is known in standard biochemical terms as Asp(B10), des(BI), des(B27-30) Human Insulin.

The insulin analogs of the present invention have a reduced propensity to dimerize or otherwise self-associate to higher molecular weight forms, either in a solution which contains zinc or a solution which is zinc-free. In that the analogs are generally monomeric in solution, a rapid onset of activity is achieved upon administration.

As mentioned hereinabove, the invention includes pharmaceutically acceptable salts of the insulin analogs. Preferred such salts are those of zinc, sodium, potassium, magnesium, calcium, or combinations of these salts.

The insulin analogs of this invention can be prepared by any of a variety of recognized peptide synthesis techniques including classical (solution) methods, solid-phase methods, semisynthetic methods and the more recently available recombinant DNA methods.

In the solid-phase technique, the amino acid sequence is constructed sequentially from an initial, insoluble, resin-supported C-terminal amino acid.

Techniques for the solid phase method are described by J. Stewart et al., Solid-Phase Peptide Synthesis, Freeman and Co., San Francisco, 1969.

In general, in the solid-phase method, the amino acid corresponding to the C-terminal amino acid residue of the desired peptide is anchored to an insoluble resin support, and the peptide chain then is formed beginning at the resin-supported C-terminal amino acid. Individual amino acids are introduced sequentially until the desired amino acid sequence is obtained. Alternatively, small peptide fragments can be prepared and introduced into the peptide chain in the desired order. The peptide chain remains attached to the resin throughout synthesis, and, upon completion of the chain the peptide is cleaved from the resin.

The peptide chain is attached to the polystyrene resin by means of an ester linkage formed between the carboxyl group of the C-terminal moiety and a specific methylene group present on the resin matrix as a site for such attachment.

The amino acids are coupled using techniques well known in the art for the formation of peptide bonds. One method involves converting the amino acid to a derivative that will render the carboxyl group more susceptible to reaction with the free N-terminal amino group of the peptide fragment. For example, the amino acid can be converted to a mixed anhydride by reaction of a protected amino acid with ethyl chloroformate, phenyl chloroformate, sec-butyl chloroformate, or isobutyl chloroformate. Alternatively, the amino acid can be converted to an active ester such as a 2,4,5-trichlorophenyl ester, a pentachlorophenyl ester, a p-nitrophenyl ester, a N-hydroxysuccinimide ester, or an ester formed from 1-hydroxybenzotriazole.

Another coupling method involves use of a suitable coupling agent, such as N,N′-dicyclohexylcarbodiimide (DCC) or N,N′-diisopropylcarbodiimide (DIC). Other appropriate coupling agents will be apparent to those skilled in the art. See Schroder and Lubke, The Peptides, Academic Press, 1965, Chapter III which is incorporated herein by reference.

It should be recognized that the α-amino group of each amino acid employed in the peptide synthesis must be protected during the coupling reaction to prevent side reactions involving the reactive α-amino function. It should also be recognized that certain amino acids contain reactive side-chain functional groups (e.g., sulfhydryl, ε-amino, β-and γ-carboxyl, imidazole, guanido and hydroxyl), and that such functional groups must also be protected both during the initial and subsequent coupling steps. Suitable protecting groups are known in the art. See for example, Protective Groups In Organic Chemistry, M. McOmie, Editor, Plenum Press, N.Y., 1973 and U.S. Patent 4,617,149 which is incorporated herein by reference.

In selecting a particular protecting group, certain conditions must be observed. An α-amino protecting group (1) must render the α-amino function inert under the conditions employed in the coupling reaction, (2) must be readily removable after the coupling reaction under conditions that will not remove side chain protecting groups and will not alter the structure of the peptide fragment, and (3) must eliminate the possibility of racemization upon activation immediately prior to coupling. A side chain protecting group (1) must render the side chain functional group inert under the conditions employed in the coupling reaction, (2) must be stable under the conditions employed in removing the α-amino protecting group, and (3) must be readily removable upon completion of the desired amino acid sequence under reaction conditions that will not alter the structure of the peptide chain.

It will be apparent to those skilled in the art that the protecting groups known to be useful for peptide synthesis will vary in reactivity to the agents employed for their removal. For example, certain protecting groups, such as triphenyl methyl and 2-(p-biphenylyl)isopropyloxycarbonyl are very labile and can be cleaved under mild acid conditions. Other protecting groups, such as t-butyloxycarbonyl, t-amyloxycarbonyl, adamantyloxycarbonyl, and p-methoxybenzyloxycarbonyl, are less labile and require moderately strong acids, such as trifluoroacetic, hydrochloric, or boron trifluoride in acetic acid, for their removal. Still other protecting groups, such as benzyloxycarbonyl, halobenzyloxycarbonyl, p-nitrobenzyloxycarbonyl, cycloalkyloxycarbonyl, and isopropyloxycarbonyl, are even less labile and require stronger acids, such as hydrogen fluoride, hydrogen bromide, or boron trifluoroacetate in trifluoroacetic acid, for their removal.

Upon completion of the desired peptide sequence, the protected peptide must be cleaved from the resin support, and all protecting groups must be removed. The cleavage reaction and removal of the protecting groups may be accomplished simultaneously or stepwise. When the resin support is a chloromethylated polystyrene resin, the bond anchoring the peptide to the resin is an ester linkage formed between the free carboxyl group of the C-terminal moiety and one of the many chloromethyl groups present on the resin matrix. It will be recognized that the anchoring bond can be cleaved by reagents which are known to be capable of breaking an ester linkage and of penetrating the resin matrix. One especially convenient method is by treatment with liquid anhydrous hydrogen fluoride. This reagent not only will cleave the peptide from the resin but will also remove all protecting groups. Hence, use of this reagent will directly afford the fully deprotected peptide. When it is desired to cleave the peptide without removing protecting groups, the protected peptide-resin can undergo methanolysis to give the protected peptide in which the C-terminal carboxyl group is methylated. The methyl ester can then be hydrolyzed under mild, alkaline conditions to give the free C-terminal carboxyl. The protecting groups on the peptide chain then can be removed by treatment with a strong acid, such as liquid hydrogen fluoride. A particularly useful technique for methanolysis is that of G. Moore et al., Peptides, Proc. 5th Amer. Pept. Symp., M. Goodman and J. Meienhofer, Eds., John Wiley, N.Y., 1977, pp. 518-521, in which the protected peptide-resin is treated with methanol and potassium cyanide in the presence of crown ether.

Another method for cleaving the protected peptide from the resin is by ammonolysis or by treatment with hydrazine. If desired, the resulting C-terminal amide or hydrazide can be hydrolyzed to the free C-terminal carboxyl, and the protecting groups can be removed conventionally.

It will also be recognized that the protecting group present on the N-terminal $\alpha$-amino group may be removed preferentially either before or simultaneous with the cleavage of the protected peptide from the resin support.

The A and B chains of the insulin analogs of the present invention can also be prepared via recombinant DNA methodology. In their preparation, a nucleotide sequence coding for the desired peptide of the A or B chain is prepared using now-routine techniques for such synthesis. These methods generally involve preparation of oligonucleotides coding both for fragments of the desired coding sequence and for the complementary sequence thereof. The oligonucleotides are designed to provide overlap of one fragment of the coding sequence with two fragments of the complementary sequence and vice versa. The oligonucleotides are paired and joined, ultimately producing the desired gene sequence.

The sequence is inserted into a cloning vector at a location which permits the peptide product for which it codes to be expressed. The construction of plasmids capable of expressing proinsulin and proinsulin analogs is described in Chance et al., European Patent Publication No 0 383 472, published August 22, 1990, the entire teaching of which is herein incorporated by reference.

The A and B chains of the insulin analogs of the present invention may also be prepared via a proinsulin-like precursor molecule using recombinant DNA techniques. See Frank et al., Peptides: Synthesis-Structure-Function, Proc. Seventh Am. Pept. Symp., Eds. D. Rich and E. Gross (1981) which is incorporated herein by reference.

The step of combining the individual A and B chains, however produced, may be achieved by the method of Chance et al., Peptides: Synthesis, Structure and Function:Proc. of Seventh American Peptide Symposium (1981) which is incorporated herein by reference.

The following examples are provided as a means of illustrating the present invention and are not to be construed as a limitation thereon.

## Example 1

### Asp(B10), des(B27-30) Human Insulin

A. Enzymatic Semisynthesis

The titled insulin analog was prepared by enzymatic semisynthesis (reverse proteolysis) using Asp(B10) desoctapeptide (B23-30) human insulin and synthetic tetrapeptide Gly-Phe-Phe-Tyr. The tetrapeptide was prepared by solid-phase peptide synthesis whereas the Asp(B10) des-octapeptide human insulin was derived from Asp(B10) human proinsulin by a trypsin hydrolysis reaction that removed sequence 23-65. The Asp(B10) proinsulin analog was prepared by recombinant DNA technology in substantial accordance with the teaching of Chance et al., European Patent Publication No. 0 383 472, published August 22, 1990, the entire teaching of which is herein incorporated by reference. Page 35 of EPO 0 383 472 discloses nucleotide linker sequence (ii) which was used to construct plasmid pRB176 encoding Met-Tyr-[Asp(B1), Lys(B28), pro(B29)HPI]. The Asp(B10) plasmid used to produce starting material for this invention comprises a linker similar to linker (ii) except the sequence CGAC encoding Asp in sequence (ii) was replaced with the sequence TTTT encoding Phe and the sequence CAT encoding His was replaced with the sequence GAC to create a plasmid encoding Asp(B10), Lys(B28), Pro(B29) human proinsulin. The redundancy of the genetic code and the knowledge of the skilled artisan allows one to make numerous substitutions to construct plasmids encoding an Asp(B10) human proinsulin.

The Asp(B10) des-octapeptide human insulin was prepared as follows: 700 ml (9.8 grams) of folded Asp(B10) human proinsulin, in a pH 2.5 glycine buffer, was adjusted to pH 10.2 by adding 25 ml of ammonium hydroxide with stirring. The pH was then quickly adjusted to 9.10 with the addition of 20 ml of concentrated hydrochloric acid. To this solution was added 7 ml of a calcium chloride solution in water (14.7 mg/ml) followed by 70 ml of a 14 mg/ml solution of porcine trypsin in water giving an enzyme to substrate ratio of about 1:10 on a weight basis. The tryptic hydrolysis reaction was mixed well, then stored at 25° for 17 hours, at which point the reaction was stopped by adding 160 ml of glacial acetic acid giving a final pH of 3.09. 105 ml of acetonitrile was then added.

For isolation and purification of the resulting Asp(B10) des-octapeptide human insulin, the tryptic digest solution was then pumped onto a 5.5 x 30 cm. C-18 Vydac HPLC column at 25°C. Two buffers were prepared for this chromatography step: buffer A, consisting of 10 parts acetonitrile and 90 parts of a 0.5% TFA (trifluoroacetic acid) solution, and buffer B, consisting of 50 parts acetonitrile and 50 parts 0.5% TFA. After briefly washing with buffer A, a linear gradient from 0-20% buffer B was pumped through the column at 2.4 ml/min. for 16 hours followed by a linear gradient of 20-70% buffer B at 8 ml/min. for 8 hours. A major peak containing the Asp(B10) des-octapeptide human insulin eluted at about the 50% buffer B level. Fractions containing the present material based on analytical reverse-phase HPLC were combined and lyophilized. Final weight recovery was 5.5 grams with an HPLC purity of 93%. The authenticity of the Asp(B10) desoctapeptide (B23-30) human insulin was verified by amino acid analysis, mass spectroscopy, and $NH_2$-terminal analysis.

For the preparation of the titled insulin analog, 545 mg of Asp(B10) des-octapeptide human insulin and 300 mg of synthetic tetrapeptide Gly-Phe-Phe-Tyr were combined in 15 ml of a solution containing one part dimethyl sulfoxide, two parts 1,4-butanediol, and one part 0.35 M tris buffer at 37°C (pH not adjusted). Porcine trypsin (85 mg) was added. The solution was mixed well and stirred occasionally for 360 minutes at 37°C. The proteins were precipitated with cold acetone, washed with diethyl ether, dried with a stream of nitrogen, and finally dissolved in 75 ml of 6 M guanidine-HCl (in 0.01 N HCl).

This clear sample solution was gel filtered on a 5 x 200 cm column of Sephadex G-50 (Superfine) and eluted with 1 M acetic acid at 4°C. The effluent was monitored at 276 nm and by reverse-phase HPLC. Fractions containing the titled product were pooled (~275 ml), diluted to 500 ml with water, pumped onto a 21 x 250 mm C-8 Zorbax column, and the products eluted in an acetonitrile gradient in 0.1 M sodium phosphate, pH 2 buffer.

The appropriate fractions containing the titled insulin analog, as determined by analytical HPLC, were pooled, diluted four-fold with water, and pumped onto a 25 x 300 mm C-18 Vydac column. The desalted protein was eluted with an acetonitrile gradient in 0.5% trifluoroacetic acid. Fractions containing the purified insulin analog were pooled and lyophilized to yield 22 mg. The structure was verified by Fast Atom Bombardment Mass Spectroscopy (FAB/MS) and amino acid analysis. Results of the FAB/MS gave a molecular weight of 5358.3 (Theory: 5358.0). The amino acid composition was as follows based upon aspartic acid as molar unity (theoretical amino acid ratios in parenthesis): Asp, 4.00(4); Thr, 1.00(1); Ser, 2.50(3); Glu, 6.90(7); Gly, 3.92(4); Ala, 1.01(1); 1/2 Cys, 4.92(6); Val, 3.55(4): Ile, 1.73(2); Leu, 6.03(6); Tyr, 3.70(4); Phe, 2.86(3); His, 1.31(1); Arg, 1.00(1).

B. Chain Combination

1. Preparation of Asp(B10), des(B27-30) Human Insulin B-Chain

An Applied Biosystems 430A peptide synthesizer (including software revision 1.4) was used to prepare a crude peptidyl resin. 0.5 Millimoles (mMol) of the starting solid phase resin (t-BOC-Thr (Bzl) OCH$_2$ Pam resin) was used (.61 mMol/g x .91 g). All amino acids used were BOC protected and, except for glutamic acid, aspartic acid, and histidine, all were used directly as received (i.e., in cartridges from Applied Biosystems, Inc.; each cartridge contained approximately 2 mMol of protected amino acid). Glutamic acid, aspartic acid, and histidine were obtained from commercial sources and transferred to cartridges such that each cartridge contained approximately 2 mMol of the desired protected amino acid. The crude peptidyl resin was dried under vacuum at room temperature for 5 hours and its weight compared to the starting weight to assure reasonable weight gain. A small portion of sample was submitted for amino acid analysis to ensure that the desired amino acids were added in the correct proportions.

The peptide was cleaved from the peptidyl resin and side-chain deprotected by stirring for approximately 1 hour at 0°C in a solution of 10 parts (v/w) HF (containing 5% v/v p-thiocresol and 5% v/v m-cresol) to 1 part peptidyl resin. After removal of most of the HF by vacuum the peptide was precipitated in ethyl ether. After several rinses with ethyl ether followed by vacuum filtration, the peptide was dissolved in approximately 200 ml of 8 M guanidine-HCl, pH 11, containing 0.1 M tris, 35 mg/ml Na$_2$SO$_3$ and 25 mg/ml Na$_2$S$_4$O$_6$. The solution was adjusted to pH 8.8 with 5 N NaOH and allowed to stir vigorously for 3 hours at room temperature.

The resulting S-sulfonated peptide solution was loaded onto a 5 x 215 cm column of Sephadex G-25 (Coarse) at room temperature. The sample was eluted at 21 ml/min at room temperature using 50 mM NH$_4$HC0$_3$. The effluent was monitored at 276 nm and a pool of the desired effluent, 1000 ml, was made, frozen, and lyophilized.

2. Combination of Asp(B10) des(B27-30) Human Insulin B-Chain with Human Insulin A-Chain

The combination of the A and B chains was accomplished by the procedure of Chance et al. supra using recombinantly produced A-chain as described in EPO 0 383,472 supra. 2.27 g of the recombinant DNA-derived A-Chain S-sulfonate and 511 mg of the synthetic Asp(B10) des(27-30) B-chain S-sulfonate were combined in a flask and dissolved in 245 ml 0.1 M glycine at ambient temperature. The pH of the solution was adjusted to 10.5 with 5N NaOH, and cooled to 4°C. A dithiothreitol (DTT) solution at 15.5 mg/ml was prepared in 0.1 M glycine buffer at ambient temperature, adjusted to pH 10.5 with 5 N NaOH and then cooled to 4°C.

18.5 ml of the DTT solution were quickly added to the solution of combined A-chain and B-chain S-sulfonates (SH/SSO$^-_3$ = 1.0). The reaction solution was stirred at 4°C in a 500 ml Ehrlenmeyer flask for 26 hours. Phosphoric acid (3.7 ml) was added, dropping the pH of the reaction to 2.9.

The resulting precipitated mixture was diluted with milli-Q water and acetonitrile and purified by reversed-phase HPLC (using a 50 x 300 mm Vydac C-18 column eluted at room temperature, 2.6 ml/min., using a linear gradient of increasing acetonitrile in 0.1 M NaH$_2$PO$_4$, pH 2.3). The effluent was monitored at 280 nm. Selected fractions were assayed by analytical HPLC. Desired fractions were pooled (405 ml), diluted with 400 ml milli-Q water, combined with 1.2 g Na$_2$S$_4$O$_6$, adjusted to pH 7 with 5 N NaOH, and further purified by reversed-phase HPLC (using a 21 x 250 mm Dupont Zorbax C-8 column eluted at room temperature, 2.0 ml/min., and a linear gradient of increasing acetonitrile in 0.1 M (NH$_4$)$_2$HP0$_4$). The effluent was monitored at 280 nm. Selected fractions were assayed by analytical HPLC. Desired fractions were pooled, adjusted to pH 2.3 with H$_3$P0$_4$, and loaded onto a Vydac C-18 HPLC column (21 x 250 mm) and eluted with a linear gradient of increasing acetonitrile in 0.5% aqueous TFA. The effluent was monitored at 280 nm. Selected fractions were assayed by analytical HPLC. The appropriate fractions were pooled and lyophilized giving a yield of 135 mg of the insulin analog of greater than 91% purity by reversed-phase HPLC. The structure was verified by Fast Atom Bombardment Mass Spectrometry (FAB/MS) and by amino acid analysis. Results of the FAB/MS gave a molecular weight of 5358.5 (Theory: 5358.0). The amino acid composition was as follows based on aspartic acid as molar unity (theoretical amino acid ratios in parentheses): Asp, 4.00 (4); Thr, 0.98 (1); Ser, 2.72 (3); Glu, 7.14 (7); Gly, 3.93 (4); Ala, 0.98 (1); 1/2 Cys, 5.44 (6); Val, 3.44 (4); Ile, 1.51 (2); Leu, 6.14 (6); Tyr, 3.86 (4); Phe, 2.88 (3); His, 1.09 (1); Arg, 0.97 (1).

## Example 2

### Asp(B10), des(B28-30) Human Insulin

The titled insulin analog was prepared by enzymatic semisynthesis (reverse proteolysis) per Example 1 using Asp(B10) des-octapeptide (B23-30) human insulin and synthetic pentapeptide Gly-Phe-Phe-Tyr-Thr. The pentapeptide was prepared by solid-phase peptide synthesis whereas the Asp(B10) des-octapeptide human insulin was derived from Asp(B10) human proinsulin by a trypsin hydrolysis reaction that removed sequence 23-65 as described in Example 1.

For preparation of the titled insulin analog, 765 mg Asp(B10) des-octapeptide human insulin and 500 mg of synthetic pentapeptide Gly-Phe-Phe-Tyr-Thr were combined in 21 ml of a solution containing one part dimethylsulfoxide, two parts 1,4-butanediol, and one part 0.35 M tris buffer at 37°C (pH not adjusted). Porcine trypsin (127 mg) was added. The solution was mixed well and stirred occasionally for 240 minutes at 37°C. The proteins were precipitated with cold acetone, washed with diethyl ether, dried with a stream of nitrogen, and finally dissolved in 50 ml of 6 M guanidine-HCl (made with 0.01 N HCl). This clear sample solution was gel filtered on a 5 x 200 cm column of Sephadex G-50 (Superfine) eluted with 1 M acetic acid at 4°C. The effluent was monitored at 276 nm and by reverse-phase HPLC. Fractions containing the titled product were pooled (~250 ml), diluted to 500 ml with water, pumped onto a 21 x 250 mm C-8 Zorbax column, and the products eluted in an acetonitrile gradient in 0.1 M sodium phosphate, pH 2 buffer. The appropriate fractions containing the titled insulin analog, as determined by analytical HPLC, were pooled, diluted five-fold with water (400 ml), and pumped onto a 25 x 300 mm C-18 Vydac column. The desalted protein was eluted with an acetonitrile gradient in 0.5% trifluoroacetic acid. Fractions containing the purified insulin analog were pooled and lyophilized to yield 47 mg. The structure was verified by Fast Atom Bombardment Mass Spectrometry (FAB/MS) and amino acid analysis. Results of the FAB/MS gave a molecular weight of 5459.2 (Theory: 5459.1). The amino acid composition was as follows based on aspartic acid as molar unity (theoretical amino acid ratios in parentheses): Asp, 4.00(4); Thr, 1.86(2); Ser, 2.56(3); Glu, 7.03(7); Gly, 4.01(4); Ala, 1.07(1); 1/2 Cys, 5.38(6); Val, 3.76(4); Ile, 1.85(2); Leu, 6.14(6); Tyr, 3.80(4); Phe, 2.94(3); His, 1.15(1); Arg, 0.96(1).

## Example 3

### Solution Stability

Degradation analysis was performed by incubating samples of insulin or insulin analogs at 50°C at concentrations of approximately 3.5 mg/ml in 0.1 M sodium phosphate, pH 7.4 (unless otherwise noted), 0.25% cresol and 1.6% glycerol. At varying times, portions of the incubation mixture were quenched with a 10-fold excess of 6 M guanidine-HCl, 0.1 M sodium phosphate, pH 3.0, 23°C until analyzed by HPLC size exclusion. The quenched incubation reactions were stored at 5°C for up to two weeks without alterations in the HPLC size-exclusion properties. HPLC size-exclusion analysis was done using a DuPont Zorbax GF-250 column (50 µl injections) with 6 M GdnHCl, 0.1 M sodium phosphate as the eluting solution (flow rate = 1 ml/min, 23°C, and detection at 278 nm). The rate of monomer degradation was determined from the slope of the log of the area of the monomer peak versus time and the half-life (t 1/2) for degradation was determined according to: t 1/2 = 0.693 ÷ rate. Results are shown in Table 1.

8

## Table 1

## Solution Stability

| | t 1/2 Monomer (days at 50°C (+/- 10%)) | |
|---|---|---|
| Molecule | pH 7.4 | pH 6.1 |
| BHI + Zinc | 94 | |
| Asp(B10), des(B27-30)HI | 89 | |
| des(B29-30)HI | 49 | |
| Asp(B10), des(B30)HI | 49 | |
| des (B27-30)HI | 46 | |
| Asp(B10), des(B28-30)HI | 37 | 193 |
| Asp(B10)HI | 33 | |
| des(B28-30)HI | 32 | |
| des(B30)HI | 24 | |
| BHI (Zinc-free) | 12 | |
| des(B26-30)HI | IN[a] | |
| Asp(B10), des(B26-30)HI | IN[a] | |

[a]Compounds would not remain soluble throughout time course of experiment

Example 4

Equilibrium Denaturation

Equilibrium denaturation experiments were performed according to the teaching of Brems et al, (1990) Biochemistry 29:9289-9293, the entire teaching of which is incorporated herein by reference. Protein stock solutions were prepared at about 10 mg/ml insulin or analog, 20 mM HEPES, pH 7.5, 20% (v/v) ethanol and 1 mM EDTA with or without GdnHCl. Denaturant stocks were prepared at about 7 M GdnHCl, 20 mM Hepes, pH 7.5, 20% (v/v) ethanol and 1 mM EDTA. Denaturation samples were prepared by mixing different ratios of protein and denaturant stock solutions and a solution of 20 mM HEPES, pH 7.5, 20% (v/v) ethanol, and 1 mM EDTA to obtain the desired denaturant concentration. GdnHCl concentrations were determined by refractometry using an Abbe-3L refractometer.

The protein stock solutions were added last when preparing the denaturation samples, then the entire sample was gently mixed by swirling. GdnHCl has a maximum solubility of about 7 M in aqueous solutions of 20% ethanol, therefore samples with greater than 7 M were made from a stock GdNHCl solution of about 8 M that did not contain ethanol. Circular dichroism measurements were made with an Aviv 62D spectrophotomer at 23°C. Results are shown in Tables II and III.

## Table II

### EQUILIBRIUM DENATURATION

|     |                             | Delta G (+/- 6%) |
| --- | --------------------------- | ---------------- |
| 1.  | Asp(B10), des(B27-30)HI     | 6.2              |
| 2.  | Asp(B10), des(B29-30)HI     | 6.0              |
| 3.  | Asp(B10), des(B26-30)HI     | 5.8              |
| 4.  | Asp(B10), des(B30)HI        | 5.5              |
| 5.  | Asp(B10), des(B28-30)HI     | 5.4              |
| 6.  | Asp(B10)HI                  | 5.1              |
| 7.  | BHI (Zinc-free)             | 5.1              |
| 8.  | Asp(B10) des(B23-30)HI      | 5.1              |
| 9.  | des(B28-30)HI               | 5.0              |
| 10. | des(B30)HI                  | 4.6              |
| 11. | des(B26-30)HI               | 4.5              |
| 12. | des(B27-30)HI               | 4.3              |
| 13. | des(B29-30)HI               | 4.3              |
| 14. | des(B23-30)HI               | 3.8              |

## Table III

### EQUILIBRIUM DENATURATION

|     |                             | Mid-Point (+/- 2%) |
| --- | --------------------------- | ------------------ |
| 1.  | Asp(B10), des(B27-30)HI     | 5.8                |
| 2.  | Asp(B10), des(B26-30)HI     | 5.7                |
| 3.  | Asp(B10), des(B30)HI        | 5.6                |
| 4.  | Asp(B10) des(B23-30)HI      | 5.6                |
| 5.  | Asp(B10), des(B29-30)HI     | 5.5                |
| 6.  | Asp(B10), des(B28-30)HI     | 5.5                |
| 7.  | Asp(B10)HI                  | 5.3                |
| 8.  | des(B29-30)HI               | 5.2                |
| 9.  | des(B27-30HI                | 5.1                |
| 10. | des(B26-30)HI               | 5.1                |
| 11. | des(B30)HI                  | 5.0                |
| 12. | des(B28-30)HI               | 5.0                |
| 13. | des(B23-30)HI               | 4.7                |
| 14. | BHI (Zinc-free)             | 4.5                |

### Example 5

Equilibrium Ultracentrifugation

The aggregation behavior of the insulin was determined by sedimentation equilibrium ultracentrifugation. The association state of each analog was measured using the techniques described in Pekar, A.H. and Frank, B.H. (1972) Biochemistry 11:4013-4016, the entire teaching of which is herein incorporated by reference. The results are given in Table IV for the insulin and the related compounds at protein concentrations of 3.5 mg/ml, pH 7.2, 0.05 M NaCl- 0.05 M sodium phosphate and 22°C. The values given are the observed weight average molecular weight divided by the monomer molecular weight of the compound. Therefore this ratio is an index of the degree of aggregation of the insulin analogs. The result is also given for zinc-free BHI. Obviously the compounds of interest exhibit very weak self-association behavior in comparison to human insulin.

## Table IV

### Equilibrium Ultracentrifugation

| | Compound | Mw/M1 |
|---|---|---|
| 1. | Asp(B10), Des(B27-30)HI | 1.06 |
| 2. | Asp(B10), Des(B28-30)HI | 1.20 |
| 3. | Des(B27-30)HI | 1.80 |
| 4. | Asp(B10), Des(B29-30)HI | 2.0 |
| 5. | Asp(B10)HI | 2.3 |
| 6. | Des(B28-30)HI | 2.3 |
| 7. | Des(B29-30)HI | 4.6 |
| 8. | BHI (Zinc-free) | 6.6 |

### Example 6

Receptor Binding

The physiological effects of the insulin analogs of the present invention were shown in the following in vitro insulin receptor binding assay. Microsomal membranes were obtained from full-term human placenta by published methods (e.g., Marshall et al. (1974) Characterization of the Insulin and Somatomedin-C Receptors in Human Placental Cell Membranes, J. Clin. Endocrin. Metab., 39:283-292). Approximately 30 $\mu$g of protein was incubated with 45,000-50,000 cpm of $^{125}$I[A14]-human insulin and increasing doses of cold competing ligand in 0.5 ml of 100 mM Hepes, pH 7.8, 120 mM NaCl, 5 mM KCl, 1.2 mM $MgSO_4$, 8 mM glucose and 0.25% BSA. After 24 hours at 4°C, the membranes were collected on glass fiber filters using a Skatron Cell Harvester and the bound $^{125}$I was determined. For comparison of the various insulin analogs with a human insulin standard (see data in Table V) competitive binding curves were constructed to determine effective hormone concentrations ($ED_{50}$) for 50% displacement of $^{125}$I insulin. Asp(B10), des(B27-30) human insulin and Asp(B10), des(B28-30) human insulin were especially potent in these receptor binding studies.

## Table V

## Relative Binding Potency to Human Insulin Receptor[a]

| Compound | Mean ± S.E.M. |
|---|---|
| Human Insulin (HI) | 100% (n = 2) |
| Asp(B10) HI | 175 ± 17% (n = 2) |
| des(B28-30) HI | 69 ± 2% (n = 3) |
| des(B27-30)HI | 125 ± 14% (n = 2) |
| Asp(B10), des(B28-30)HI | 309 ± 19% (n = 2) |
| Asp (B10), des(B27-30)HI | 275 ± 15% (n = 2) |
| Asp (B10), des(B26-30)HI | 172 ± 95 (n = 2) |

[a]$EC_{50}$ for human insulin was 0.29 nM

### Example 7

### Rat Studies

The physiological effects of the insulin analogs of the present invention were shown in the following in vivo assay system.

Normal male Sprague Dawley rats from the Charles River Laboratories (Portage, MI) were used as test animals. They were obtained at a weight range of 160-180 gms and maintained for one week in animal rooms at 75°F with a controlled light cycle (lights on 7:00 a.m. - 7:00 p.m., lights off 7:00 p.m. - 7:00 a.m.) The animals were fed Purina rat chow 5001 ad libitum. Rats used for each assay were fasted for 16 hours before being used. They weighed about 200 gms when first used. On reaching a fasted weight of about 275 gm (over a period of three weeks), the animals were no longer used. One group of ten male rats was used each day for each compound tested (i.e., biosynthetic human insulin, porcine insulin and human insulin analog). Each group was used only once during the week. The ten rats were divided into two groups of five rats each. One group served as control and was given vehicle alone subcutaneously. The other group of five rats was given the compound to be tested. The proteins were dissolved in 0.05 N HCl (pH 1.6) to provide a stock solution of 100 μgm per ml. From this, a number of dilutions were made in normal saline which was injected subcutaneously into the rats. A 100 μl sample of blood was taken from the tail vein of each rat at zero time and 30 minutes, 1 hour, 2 hours, 3 hours and 4 hours after administration. Glucose was determined colorimetrically by a glucose oxidase method (Sigma Chemical Co.). The percent change in blood glucose from the zero time value was calculated for each rat and the final results were expressed as the mean percent change ± SEM in the experimental group corrected for the mean change in the control group for that day.

A dose-response curve was drawn from tests with 4 to 7 different concentrations of the compound tested using the maximal response at a given time (usually one or two hours after administration of the protein). From this curve an $ED_{50}$ value was determined as that subcutaneous dose (μg/kg) of the protein which gave half the maximal hypoglycemic response. The results are shown in Table VI following.

## Table VI[a]

| Compound | ED$_{50}$[b] | Relative Hypoglycemic Activity(%)[c] |
|---|---|---|
| Human Insulin (HI)[d] | 7.75 | 100 |
| Asp(B10) HI | 9.8 | 79 |
| des(B28-30) HI | 8.1 | 96 |
| des(B27-30)HI | 12.7 | 61 |
| Asp(B10), des(B28-30) HI | 7.7 | 101 |
| Asp(B10), des(B27-30) HI | 11.9 | 65 |
| Asp(B10), des(B26-30) HI | 11.0 | 70 |

[a]All values shown refer to blood samples taken one hour after administration of the compound.

[b]Expressed in μg/kg, subcutaneous

[c]Relative to human insulin

[d]Biosynthetic human insulin

As noted previously, the insulin analogs of the present invention have a reduced propensity to dimerize or otherwise self-associate to higher molecular weight forms. Thus, upon administration of one or more of said analogs, a rapid onset of activity is achieved. The insulin analogs of the present invention are effective in treating hyperglycemia by administering to a patient in need thereof an effective amount of an insulin analog of formula 1. As used herein the term "effective amount" refers to that amount of one or more insulin analogs of the present invention needed to lower or maintain blood sugar levels either therapeutically or prophylactically. This amount typically may range from about 10 units up to about 60 units or more per day (or about 0.3 to about 2 mg assuming approximately 29 units per mg). However, it is to be understood that the amount of the insulin analog(s) actually administered will be determined by a physician in light of the relevant circumstances including the condition being treated (i.e., the cause of the hyperglycemia) the particular analog to be administered, the chosen parenteral route of administration, the age, weight and response of the individual patient and the severity of the patient's symptoms. Therefore, the above dosage ranges are not intended to limit the scope of the invention in any manner.

The insulin analogs of the invention are administered to a patient in need thereof (i.e., a patient suffering from hyperglycemia) by means of pharmaceutical compositions containing an effective amount of at least one insulin analog of formula I in combination with one or more pharmaceutically acceptable excipients or carriers. For these purposes, the pharmaceutical compositions may typically be formulated so as to contain about 100 units per ml or similar concentrations containing an effective amount of the insulin analog(s). These compositions are typically, though not necessarily, parenteral in nature and may be prepared by any of a variety of techniques using conventional excipients or carriers for parenteral products which are well known in the art. See, for example, Remington's Pharmaceutical Sciences, 17th Edition, Mack Publishing Company, Easton, PA, USA (1985) which is incorporated herein by reference. For example, dosage forms for parenteral administration may be prepared by suspending or dissolving the desired amount of at least one insulin analog of formula I in a nontoxic liquid vehicle suitable for injection such as an aqueous medium and sterilizing the suspension or solution. An accompanying vial or vehicle can be provided for purposes of mixing prior to administration. Pharmaceutical compositions adapted for parenteral administration employ diluents, excipients and carriers such as water and water-miscible organic solvents such as glycerin, sesame oil, groundnut oil, aqueous propylene glycol, N,N'-dimethylformamide and the like. Examples of such pharmaceutical compositions include sterile, isotonic, aqueous saline solutions of the insulin analogs of formula I which can be buffered with a pharmaceutically acceptable buffer and which are pyrogen free. Additionally, the parenteral pharmaceutical formulation may contain preservatives such as phenol or meta-cresol. Agents to adjust pH of the final product such as sodium hydroxide or hydrochloric acid may also be used.

The insulin analogs of the present invention may also be formulated into pharmaceutical compositions suitable for intranasal administration. Such compositions are disclosed in detail in European Patent Application 0200383 A3 which is incorporated herein by reference. Briefly, such compositions are formulated with one or more pharmaceutically acceptable diluents, a pharmaceutically acceptable amount of an alkali metal salt, the ammonium salt, or the free acid of a substantially zinc-free insulin, and optionally, an absorption enhancing amount of at least one absorption enhancing agent selected from the group consisting of (1) oleic acid or an ester or salt thereof, (2) a liquid form sorbitan fatty acid ester, (3) a liquid form polyoxyethylene derivative of a sorbitan fatty acid ester, and (4) a liquid form hydroxypolyoxyethylene-polyoxypropylene-polyoxyethylene copolymer.

## Example VIII

## Dog Studies

To demonstrate the efficacy and rapid action of subcutaneous administration of the insulin analogs Asp(B10), des(B27-30) human insulin and Asp(B10), des(B28-30) human insulin versus a regular soluble human insulin formulation (Humulin R®) the following study was conducted. Male beagle dogs weighing 10 to 12 kg were maintained in excellent physical condition prior to and during the course of the study. The dogs were fasted for 16 hours but had access to water to ensure against unexpected fluctuations in insulin levels. During the entire course of the study the dogs were anesthetized with sodium pentobarbitol and their body temperature maintained with heating pads to minimize glucose fluctuation. The insulin test samples (i.e., Asp(B10), des(B27-30) human insulin and Asp(B10), des(B28-30) human insulin) were formulated at 3.5 mg/ml in the same diluent used for Humulin R®. In these studies the hypoglycemic potency for each insulin analog was assumed equal to insulin on a weight basis; therefore these test solutions were considered to contain 100 units/ml. All insulin test solutions were administered subcutaneously by injecting 0.1 unit per kg beneath the epidermis of the outer side of the flank of each of eight or ten dogs depending on the sample. Blood samples were drawn at 30, 15 and 0 minutes before administration and at 10, 20, 30, 45, 60, 90, 120, 180, and 240 minutes after administration. Glucose was determined colorimetrically by a glucose oxidase method. The results of this comparative study are shown in Table VII.

## Table VII

## Blood Glucose Levels: % of 0 Time[a]

| Time (Min) | Humulin R® (n = 8) | Asp(B10) des(B27-30) HI (n = 8) | Asp(B10) des(B28-30) HI (n = 10) |
|---|---|---|---|
| -30 | 102 ± 1.6 | 92 ± 2.1 | 103 ± 2.9 |
| -15 | 101 ± 1.6 | 99 ± 1.2 | 101 ± 2.0 |
| 0 | 100 | 100 | 100 |
| 10 | 100 ± 1.3 | 93 ± 1.5 | 98 ± 1.8 |
| 20 | 99 ± 1.9 | 80 ± 3.3 | 93 ± 2.0 |
| 30 | 97 ± 2.1 | 63 ± 3.1 | 81 ± 2.9 |
| 45 | 86 ± 4.1 | 47 ± 4.1 | 67 ± 4.4 |
| 60 | 79 ± 4.9 | 43 ± 3.4 | 60 ± 4.8 |
| 90 | 67 ± 4.6 | 48 ± 2.9 | 67 ± 5.7 |
| 120 | 64 ± 4.8 | 58 ± 4.1 | 73 ± 4.9 |
| 180 | 62 ± 5.0 | 87 ± 2.6 | 89 ± 1.9 |
| 240 | 77 ± 4.9 | 89 ± 2.0 | 90 ± 2.2 |

[a]Blood glucose levels: mean ± S.E.M.

**Claims**

1. An insulin analog of the formula SEQ ID NO:1 properly cross-linked to SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

2. The insulin analog of Claim 1 wherein Xaa at position 21 of SEQ ID NO:1 is Asn.

3. The insulin analog of Claim 2 wherein Xaa at position 1 of SEQ ID NO:2 is Phe.

4. The insulin analog of Claim 3 wherein Xaa at position 3 of SEQ ID NO:2 is Asn.

5. An insulin analog of the formula SEQ ID NO:1 properly cross-linked to amino acid residues 1 to 26 of SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

6. The insulin analog of Claim 5 wherein Xaa at position 21 of SEQ ID NO:1 is Asn.

7. The insulin analog of Claim 6 wherein Xaa at position 1 of SEQ ID NO:2 is Phe.

8. The insulin analog of Claim 7 wherein Xaa at position 3 of SEQ ID NO:2 is Asn.

9. An insulin analog of the formula SEQ ID NO:1 properly cross-linked to amino acid residues 2 to 27 of SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

10. An insulin analog of the formula SEQ ID NO:1 properly cross-linked to amino acid residues 2 to 26 of SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

11. An insulin analog of the formula SEQ ID NO:1 properly cross-linked to SEQ ID NO:2 or a pharmaceutically acceptable salt thereof, wherein Xaa at position 21 of SEQ ID NO:1is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn, for use as an antihyperglycemic agent.

12. The analog of Claim 11 wherein Xaa at position 21 of SEQ ID NO:1 is Asn, Xaa at position 1 of SEQ ID NO: 2 is Phe and Xaa at position 3 of SEQ ID NO:2 is Asn for use as an antihyperglycemic agent.

13. An insulin analog of the formula SEQ ID NO:1 properly cross-linked to amino acid residues 1 to 26 of SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn for use as an antihyperglycemic agent.

14. The analog of Claim 13 wherein Xaa at position 21 of SEQ ID NO:1 is Asn, Xaa at position 1 of SEQ ID NO:2 is Phe and Xaa at position 3 of SEQ ID NO:2 is Asn, for use as an anti hyperglycemic agent.

15. A pharmaceutical formulation comprising, in a pharmaceutically acceptable diluent, an insulin analog of the formula SEQ ID NO:1 properly cross-linked to SEQ ID NO:2 or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

16. The pharmaceutical formulation of Claim 15 wherein Xaa at position 21 of SEQ ID NO:1 is Asn, Xaa at position 1 of SEQ ID NO:2 is Phe and Xaa at position 3 of SEQ ID NO:2 is Asn.

17. A pharmaceutical formulation comprising, in a pharmaceutically acceptable diluent, an insulin analog of the formula: SEQ ID NO:1 properly cross-linked to amino acid residues 1 to 26 of SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

18. The pharmaceutical formulation of Claim 17 wherein Xaa at position 21 of SEQ ID NO:1 is Asn, Xaa at position 1 of SEQ ID NO:2 is Phe and Xaa at position 3 of SEQ ID NO:2 is Asn.

19. A process for preparing an insulin analog as claimed in any on of claims 1 to 10, which comprises cross-linking a compound of SEQ ID NO:1 with a compound of SEQ ID NO:2.

20. An insulin analog whenever prepared by a process according to Claim 19.

**Claims for the following Contracting States : GR, ES**

1. A process for preparing an insulin analog which comprises properly cross-linking SEQ ID NO:1 to SEQ ID NO:2, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

2. The process according to Claim 1 wherein Xaa at position 21 of SEQ ID NO:1 is Asn.

3. The process according to Claim 2 wherein Xaa at position 1 of SEQ ID NO:2 is Phe.

4. The process according to Claim 3 wherein Xaa at position 3 of SEQ ID NO:2 is Asn.

5. A process for preparing an insulin analog which comprises properly cross-linking SEQ ID NO:1 to amino acid residues 1 to 26 of SEQ ID NO:2, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, and Xaa at position 3 of SEQ ID NO:2 is selected from the group consisting of Asp or Asn.

6. The process according to Claim 5 wherein Xaa at position 21 of SEQ ID NO:1 is Asn.

7. The process according to Claim 6 wherein Xaa at position 1 of SEQ ID NO:2 is Phe.

8. The process according to Claim 7 wherein Xaa at position 3 of SEQ ID NO:2 is Asn.

9. A process for preparing a pharmaceutical formulation which comprises admixing an insulin analog of any of Claims 1 through 8 with one or more pharmaceutically acceptable carriers, diluents or excipients therefor.


## SEQUENCE LISTING

(1) GENERAL INFORMATION:

    (i) APPLICANT:Eli Lilly and Company

    (ii) TITLE OF INVENTION:Insulin Analogs

    (iii) NUMBER OF SEQUENCES:2

    (iv) CORRESPONDENCE ADDRESS:

        (A) ADDRESSEE:Mr. C. Mark Hudson

        (B) STREET:Erl Wood Manor

        (C) CITY:Windlesham

        (D) STATE:Surrey GU20 6PH

        (E) COUNTRY:Grande Bretagne

(v)    ATTORNEY/AGENT INFORMATION:

    (A) NAME:Mr. C. Mark Hudson

    (B) REGISTRATION NUMBER:307

    (C) REFERENCE/DOCKET NUMBER: X-8620 EPO

(vi)  TELECOMMUNICATION INFORMATION:

    (A) TELEPHONE:0276 78441

    (B) TELEFAX:858177

(2) INFORMATION FOR SEQ ID NO:1 :

    (i) SEQUENCE CHARACTERISTICS:

    (A) LENGTH:21 amino acids

    (B) TYPE:amino acid

      (C) STRANDEDNESSS:

      (D) TOPOLOGY:linear

  (ii) MOLECULE TYPE:polypeptide

  (ix) FEATURE:

    (A) NAME/KEY:Variable Site

    (B) LOCATION:21

    (C) IDENTIFICATION METHOD:

    (D) OTHER INFORMATION:"This amino acid is either Asn, Asp, Glu, Gln, Ala, Gly or Ser."

  (xi) SEQUENCE DESCRIPTION: SEQ ID NO:1:
Gly Ile Val Glu Gln Cys Cys Thr Ser Ile Cys Ser Leu Tyr Gln
1               5               10              15

Leu Glu Asn Tyr Cys Xaa
                20

(3) INFORMATION FOR SEQ ID NO:2 :

    (i) SEQUENCE CHARACTERISTICS:

    (A) LENGTH:27 amino acids

    (B) TYPE:amino acid

        (C) STRANDEDNESSS:

        (D) TOPOLOGY:linear

(ii) MOLECULE TYPE:polypeptide


(ix) FEATURE:

        (A) NAME/KEY:Variable Site

    (B) LOCATION:1

    (C) IDENTIFICATION METHOD:

    (D) OTHER INFORMATION:"This amino acid is either Phe or Asp."

(ix) FEATURE:

        (A) NAME/KEY:Variable Site

    (B) LOCATION:3

    (C) IDENTIFICATION METHOD:

    (D) OTHER INFORMATION:"This amino acid is either Asn or Asp."

(xi) SEQUENCE DESCRIPTION: SEQ ID NO:2:
Xaa Val Xaa Gln His Leu Cys Gly Ser Asp Leu Val Glu Ala Leu
 1               5                   10                  15

Tyr Leu Val Cys Gly Glu Arg Gly Phe Phe Tyr Thr
                        20                  25